# EUROPÄISCHE PATENTSCHRIFT

(11) **EP 1 195 167 B1**
(45) Veröffentlichungstag und Bekanntmachung des Hinweises auf die Patenterteilung: **23.03.2005**
(21) Anmeldenummer: 01121977.1
(22) Anmeldetag: 13.09.2001
(51) Int. Cl.: A61K 49/22, A61K 41/00, C08G 18/48

(54) **Verwendung einer Gelmasse**
Use of a gel
Utilisation d'un gel

(30) Priorität: 09.10.2000 DE 10050114
(43) Veröffentlichungstag der Anmeldung: 10.04.2002
(73) Patentinhaber: Richard Wolf GmbH, 75438 Knittlingen (DE)
(72) Erfinder: Krauss, Werner, Dipl.-Ing., 75438 Knittlingen (DE); Bauer, Edgar, Dipl.-Ing., 76703 Kraichtal (DE); Jaggy, Peter, Dipl.-Ing., 75443 Ötisheim (DE); Benkhoff, Hermann, 37115 Duderstadt (DE)
(74) Vertreter: Wilcken, Thomas, Dipl.-Ing.

(56) Entgegenhaltungen:
- EP-A- 0 344 773
- EP-A- 0 511 570
- GB-A- 2 036 504

## Beschreibung

Die Erfindung betrifft ein akustisches Therapiegerät mit einem elektroakustischen Wandler und einem Ankoppelpolster aus Gelmasse als Koppelmedium zur Übertragung akustischer Wellen auf den Körper eines Patienten.

Lithotripsie- und Hyperthermiegeräte sind allgemein als akustische Therapiegeräte bekannt. Diese Geräte haben einen elektroakustischen Wandler als Quelle fokussierter akustischer Wellen, die von der Wandlerabstrahlfläche ausgehen sowie ein der Abstrahlfläche vorgelagertes Ankoppelpolster mit einer an der Abstrahlfläche liegenden Einkoppelfläche und einer zur Applikation am Patienten vorgesehenen Auskoppelfläche. Es ist auch möglich, gleichzeitig mit zwei oder mehr schichtweise angeordneten Koppelpolstern zu arbeiten, wenn man beispielsweise die Eindringtiefe des Wandlerfokus im Körper des Patienten im Vergleich zu der Eindringtiefe reduzieren will, die bei Anwendung nur eines Ankoppelpolsters erzielt wird. Im Übrigen werden im Falle der Lithotripsie fokussierte Stoßwellen erzeugt. Bei der Hyperthermie kommen Ultraschallwellen in Form von Dauerschall oder Schallpulsen zur Anwendung.

Die DE 195 09 004 C 1 schlägt ein Therapiegerät vor, das mit einem von verschiedenen zur Verfügung gehaltenen Ankoppelpolstern ausgestattet werden kann. Die Ankoppelpolster können jeweils austauschbar an den Wandler angeschlossen werden, indem sie beispielsweise mit ihrer Einkoppelfläche in einer Wandlerschale abgelegt und in geeigneter Weise am Wandler befestigt werden.

Ankoppelpolster sind erforderlich, um den Abstand zwischen der Abstrahlfläche des Wandlers und der Körperoberfläche des Patienten als sogenannte Vorlaufstrecke zu überbrücken, die bei Bedarf auch durch Anwendung mehrerer Koppelpolster vergrößert und variabel eingestellt werden kann (DE 33 12014 A1).

Es ist bekannt, Ankoppelpolster aus bestimmten akustischen Koppelmedien, wie Polyacrylamid-Gel oder synthetischem Gummi, als Formkörper auszubilden oder eine Polsterhülle mit einem solchen Gel zu füllen (DE 195 09 004 C1). Allerdings hat es sich gezeigt, dass solche Materialien bzw. Koppelmedien relativ hohe Schallenergieverluste mit sich bringen und auch im Hinblick auf die gewünschte hohe Schallabsorption beim Übergang von der Wandlerabstrahlfläche auf das Koppelmedium unbefriedigend sind. Außerdem wurde festgestellt, dass bekannte Gele zu Reizungen der Haut des Patienten führen können, wenn sie hiermit direkt in Kontakt gebracht werden. Schließlich ist festzustellen, dass die bisher üblichen Gele häufig nichtweichmacherfrei und nicht ausreichend UV-stabil sind, so dass es im Laufe der Zeit zu einer nachteiligen Änderung der akustischen Eigenschaften dieses Koppelmediums kommen kann.

Der Erfindung liegt daher die Aufgabe zugrunde, ein akustisches Therapiegerät mit einem Koppelmedium aus Gelmasse vorzuschlagen, die sich durch geringe Schallenergieverluste auszeichnet, eine hohe Schallabsorption beim Übergang des Ultraschalls von der Wandlerabstrahlfläche gewährleistet und einen geringen akustischen Widerstand bietet. Weitere Anforderungen an das Koppelmedium sind, dass es hautfreundlich ist, eine große Formstabilität aufweist und aus vorerwähnten Gründen weichmacherfrei und UV-stabil sein soll.

Zur Lösung dieser Aufgabe ist bei einem Therapiegerät gemäß dem Oberbegriff des Anspruchs 1 die Gelmasse auf der Basis von Reaktionsprodukten aus Polyolen und Polyisocyanaten gebildet, wobei die Polyolkomponente aus einem oder mehreren Polyolen mit Hydroxylzahlen unter 112 und gegebenenfalls anderen aus der Polyolchemie bekannten Polyolen und Zusatzstoffen besteht, die Isocyanat-Kennzahl des Reaktionsgemisches im Bereich von 15 bis 59,81 liegt und das Produkt aus Isocyanat-Funktionalität und Funktionalität der Polyolkomponente mindestens 6,15 berträgt.

Zweckmäßigerweise enthält die Gelmasse zusätzlich aus der Polyurethanchemie bekannte Füll- und/oder Zusatzstoffe in einer Menge von insgesamt bis zu 50 Gew.-%, bezogen auf das Gesamtgewicht der Gelmasse.

Unter lsocyanat-Kennzahl wird das Äquivalentverhältnis (NCO/OH) x 100 verstanden. So bedeutet z. B. eine lsocyanat-Kennzahl von 15, dass auf eine reaktive OH-Gruppe in den Polyolen 0,15 reaktive NCO-Gruppen im Polyisocyanat oder auf eine reaktive NCO-Gruppe im Polyisocyanat 6,67 reaktive OH-Gruppen in den Polyolen vorliegen. Entsprechend bedeutet eine lsocyanat-Kennzahl von 70, dass auf eine reaktive OH-Gruppe in den Polyolen 0,7 reaktive NCO-Gruppen im Polyisocyanat oder auf eine reaktive NCO-Gruppe im Polyisocyanat 1,43 reaktive NCO-Gruppen im Polyisocyanat vorliegen.

Weitere Details zur Herstellung der erfindungsgemäß zur Verwendung vorgeschlagenen Gelmasse finden sich in der EP 0 511 570 B 1, aus der eine solche Gelmasse bekannt ist, und zwar zur Verwendung in druckverteilenden Elementen in Form von Sitzpolstern und Auflagen für Körperoberflächen.

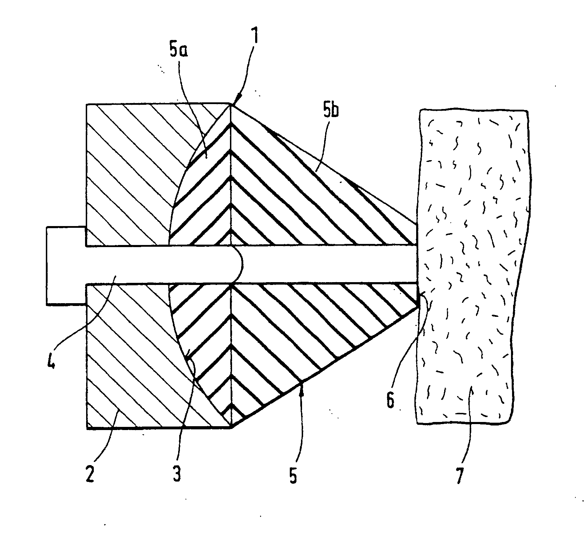

In der Zeichnung ist ein Ausführungsbeispiel der Erfindung dargestellt. Die einzige Figur zeigt schematisch und stark vereinfacht einen Schnitt durch ein akustisches Therapiegerät im Bereich seines elektroakustischen Wandlers mit einem Ankoppelpolster.

Das Therapiegerät 1 weist einen Wandler 2 als Quelle für akustische Wellen, wie beispielsweise Stoßwellen bei der Lithotripsie, auf. Der Wandler 2 hat eine konkav geformte Abstrahlfläche 3, deren Form dadurch vorgegeben ist, dass der Wandler in bekannter Weise aus einem Träger in Form einer Kugelkalotte besteht, in der piezoelektrische Wandlerelemente mosaikartig angeordnet sind, die von einer Versorgungseinheit impulsweise mit Hochspannung angesteuert werden.

Das Gerät 1 ist außer mit dem Therapiewandler 2 auch mit einem Diagnosewandler 4 ausgestattet, der als B-Scanner ausgebildet ist und über den in Form von Schnittbildern das zu beschallende Objekt und dessen Position im Verhältnis zum Wandlerfokus auf einem Monitor abgebildet werden können.

Die Übertragung der akustischen Therapiewellen auf das Gewebe des Patienten 7 erfolgt mittels eines Ankoppelpolsters 5. Dieses liegt auf der Haut des Patienten mit seiner Auskoppelfläche 6 auf. Das Ankoppelpolster kann einstückig als form-stabiler Körper aus dem als Koppelmedium vorgeschlagenen Gel bestehen. Beim dargestellten Ausführungsbeispiel wird allerdings kein einteiliges Ankoppelpolster allein verwendet. Vielmehr ist die Schale des Wandlers 1 randbündig mit Gelmasse zur Bildung eines Koppelelements 5a gefüllt, auf dem ein gesondertes Koppelpolster 5b abgelegt ist, welches gegen ein Koppelpolster mit anderen Abmessungen in Schallausbreitungsrichtung ausgetauscht werden kann, um so die Möglichkeit zu haben, die effektive Vorlaufstrecke zwischen der Abstrahlfläche 3 und der Hautoberfläche des Patienten und somit auch die Tiefe des Wandlerfokus im Patientenkörper bei Bedarf variieren zu können.

Praktische Versuche haben gezeigt, dass die erfindungsgemäß zur Verwendung als Koppelmedium ausgewählte Gelmasse die gestellte Aufgabe im Vergleich zu sonstigen zum gleichen Zweck verwendeten Gelmassen einwandfrei und besser erfüllt. Jedenfalls hat sich gezeigt, dass äußerst geringe Schallenergieverluste auftreten, dass die Gelmasse hautfreundlich ist, eine hohe UV-Beständigkeit und eine gute Formstabilität hat.

## Patentansprüche

1. Akustisches Therapiegeröt (1) mit einem elektroakustischen Wandler (2) und einem Ankoppelpolster (5) aus Gelmasse als Koppelmedium zur Übertragung akustischer Wellen auf den Körper eines Patienten (7), **dadurch gekennzeichnet, dass** die Gelmasse auf der Basis von Reaktionsprodukten aus Polyolen und Polyisocyanaten gebildet ist, wobei die Polyolkomponente aus einem oder mehreren Polyolen mit Hydroxylzahlen unter 112 und gegebenenfalls anderen aus der Polyolchemie bekannten Polyolen und Zusatzstoffen besteht, die lsocyanat-Kennzahl des Reaktionsgemisches im Bereich von 15 bis 59,81 liegt und das Produkt aus Isocyanat-Funktionalität und Funktiontionalität der Polyolkomponente mindestens 6,15 beträgt.

2. Therapiegerät nach Anspruch 1, **dadurch gekennzeichnet, dass** die Gelmasse zusätzlich aus der Polyurethanchemie bekannte Füll- und/oder Zusatzstoffe in einer Menge von insgesamt bis zu 50 Gew.-%, bezogen auf das Geamtgewicht der Gelmasse, enthält.

3. Therapiegerät nach einem der Ansprüche 1 und 2, **dadurch gekennzeichnet, dass** das Ankoppelposter (5) als einstückiger form-stabiler Körper ausgebildet ist.

4. Therapiegerät nach einem der Ansprüche 1 und 2, **dadurch gekennzeichnet, dass** das Ankoppelposter (5) aus einem Koppelelement (5a) und einem auf dem Koppelement (5a) abgelegten Koppelpolster (5b) besteht.

5. Therapiegerät nach Anspruch 4, bei dem der Wandler (2) einen Träger in Form einer mit Wandlerelementen bestücken Kugelkalotte aufweist, **dadurch gekennzeichnet, dass** die Kugelkalotte zur Bildung des Koppelelementes (5a) randbündig mit der Gelmasse gefüllt ist.

## Claims

1. An acoustic therapy apparatus (1) with an electroacoustic transducer (2) and a coupling cushion (5) of a gel mass as a coupling medium for transmitting acoustic waves onto the body of a patient (7), **characterised in that** the gel mass is formed on the basis of reaction products of polyols and polyisocyanates, wherein the polyol component consists of one or more polyols with hydroxyl numbers below 112 and where appropriate of other polyols and additives known from polyol chemistry, the isocyanate number of the reaction mixture lies in the region of 15 to 59.81, and the product of the isocyanate functionality and the functionality of the polyol component is at least 6.15.

2. A therapy apparatus according to claim 1, **characterised in that** the gel mass additionally contains fillers and/or additives known from polyurethane chemistry in a quantity of in total up to 50% by weight with respect to the total weight of the gel mass.

3. A therapy apparatus according to one of the claims 1 and 2, **characterised in that** the coupling cushion (5) is designed as a one-piece shape-stable body.

4. A therapy apparatus according to one of the claims 1 and 2, **characterised in that** the coupling cushion (5) consists of a coupling element (5a) and of a coupling cushion (5b) applied on the coupling element (5a).

5. A therapy apparatus according to claim 4, with which the transducer (2) comprises a carrier in the form of a spherical calotte equipped with transducer elements, **characterised in that** the spherical calotte for forming the coupling element (5a) is filled with the gel mass in a manner flush with the edge.

## Revendications

1. Appareil acoustique de thérapie (1) comprenant un convertisseur électroacoustique (2) et un tampon de couplage (5) en gel comme milieu de couplage pour la transmission d'ondes acoustiques au corps d'un patient (7), **caractérisé en ce que** le gel est formé sur la base de produits de réaction constitués de polyols et de polyisocyanates, le composant de polyol étant constitué d'un ou de plusieurs polyols avec des indices d'hydroxyle inférieurs à 112 et éventuellement d'autres polyols et additifs connus par la chimie des polyols, l'indice d'isocyanate du mélange de réaction se situant dans la plage de 15 à 59,81 et le produit de la fonctionnalité de l'isocyanate et de la fonctionnalité du composant de polyol étant d'au moins 6,15.

2. Appareil de thérapie selon la revendication 1, **caractérisé en ce que** le gel contient en supplément des matières de charge et/ou des additifs connus par la chimie du polyuréthane, en une quantité globale allant jusqu'à 50 % en poids, rapportée au poids total de la masse de gel.

3. Appareil de thérapie selon l'une des revendications 1 et 2, **caractérisé en ce que** le tampon de couplage (5) est conçu comme un corps de forme stable en une seule pièce.

4. Appareil de thérapie selon l'une des revendications 1 et 2, **caractérisé en ce que** le tampon de couplage (5) se compose d'un élément de couplage (5a) et d'un tampon de couplage (5b) posé sur l'élément de couplage (5a).

5. Appareil de thérapie selon la revendication 4, sur lequel le convertisseur (2) présente un support sous la forme d'une calotte sphérique équipée d'éléments de convertisseur, **caractérisé en ce que** la calotte sphérique est remplie à ras bord avec le gel pour la formation de l'élément de couplage (5a).
